**Europäisches Patentamt**

**European Patent Office**

**Office européen des brevets**

(19)

(11) Veröffentlichungsnummer: **0 176 772**

**B1**

(12)

# EUROPÄISCHE PATENTSCHRIFT

(45) Veröffentlichungstag der Patentschrift:
**29.03.89**

(21) Anmeldenummer: **85110913.2**

(22) Anmeldetag: **29.08.85**

(51) Int. Cl.⁴: **A 61 K 31/375,** A 61 K 9/22, A 61 K 9/52, A 61 K 47/00 // (A61K31/375, 31:355)

(54) **Verfahren zur Retardierung von Vitamin C und E.**

(30) Priorität: **30.08.84 DE 3431825**

(43) Veröffentlichungstag der Anmeldung:
**09.04.86 Patentblatt 86/15**

(45) Bekanntmachung des Hinweises auf die Patenterteilung:
**29.03.89 Patentblatt 89/13**

(84) Benannte Vertragsstaaten:
**AT BE CH DE FR GB IT LI LU NL SE**

(56) Entgegenhaltungen:
**EP-A-0 127 297**
**FR-A-1 261 164**
**FR-A-1 446 273**

(73) Patentinhaber: **RODISMA Pharmazeutische Produkte GmbH, Siebengebirgsallee 2, D-5000 Köln 41 (DE)**

(72) Erfinder: **Roshdy, Ismail, Dr., Siebengebirgsapotheke Siebengebirgsallee 2, D-5000 Köln 41 (DE)**

(74) Vertreter: **Werner, Hans- Karsten, Dr., Deichmannhaus am Hauptbahnhof, D-5000 Köln 1 (DE)**

**Beschreibung**

Die vorliegende Erfindung betrifft ein Verfahren zur Retardierung von Vitamin C und E.

Es ist bekannt, daß Vitamin C oxydationshemmenden Einfluß haben kann (vgl. Mohler, Ascorbinsäure als Antioxidans oder als Synergist in Antioxidantien, Vorträge und Diskussionen des 11. Symposions in Mainz vom 2. bis 3. April 1964, Dr. Dietrich Steinkopf Verlag, Darmstadt 1965, Seiten 280 ff). Ferner ist bekannt, daß Vitamin C die Bildung von Nitrosaminen in Mensch und Tier sowie in Lebensmitteln verhindern kann (Sidney S. Mirvish, Ann. N.Y. Acad. Sci. 258, 1975, Seiten 180 bis 185, Jerome J. Kamm, T. Dashman, A.H. Conney, J.J. Burns, Ann. N.Y. Acad. Sci. 258, 1975, Seiten 169 bis 174, D. Schmähl. Münch. Med. WSchr. 121, 1979, Nr. 6).

Es wurde ferner nachgewiesen, daß Vitamin C durch seine reduzierenden Eigenschaften die Bildung der carzinogenen Nitrosamine aus Amin und Nitrit verhindert (B. Bertram, Deutsche Apothekerzeitung, 121. Jahrgang, Nr. 45, vom 05.11.1981, R. Schmähl, Med. Tribune, Ausgabe Deutschland 15, Nr. 41, 91/1980). Darüber hinaus wurde untersucht, ob Ascorbinsäure das Tumorwachstum beeinflussen kann (H. Kasper, Leber, Magen, Darm 10, Nr. 5, 1980, Seiten 265 bis 268). Nach Kübler und Gehler (J. Ind. J. Vit. Res. 1970, 40, Seite 442) ist bei Einzeldosen bis 180 mg Vitamin C mit einer dosisproportionalen Resorption von etwa 70 % zu rechnen. Bei einer oralen Ascorbinsäurebelastung mit 12 g werden aber nur noch 16 % resorbiert.

Nach Hornig und Moser, Vitamin C, Herausgeber J.N. Counsill und D.H. Hornig, Pl. Science Publishers, London, New Jersey, werden unter Berücksichtigung eines 3-Kompartiment-Modells bei einer Einzeldosis von 1 g sogar noch 75 % Vitamin C resorbiert.

Es ist weiterhin bekannt, daß Vitamin C und E in einem synergistischen Verhältnis zueinander stehen. So erhöht die Einnahme von Vitamin E den Gehalt an Vitamin C im Plasma. Das gleiche gilt für Vitamin C bei der Einnahme von Vitamin E (Robert. E. Keith, Ph.D., The American Journal of Clinical Nutrition 33, 1980, Seiten 2394 bis 2400). Es wurde ferner festgestellt, daß die Einnahme von 400 bis 4000 mg Vitamin C pro Tag die Menge von Mutagenen reduziert. Die Einnahme von Vitamin E in einer Menge von 120, 400 und 1200 mg pro Tag zeigt den gleichen Effekt. Durch die Kombination von beiden Vitaminen in Mengen von 400 mg pro Tag wurde die Menge an Mutagenen nochmals um 25 % reduziert (P.W. Dion et al., Proc. Amer. Assoc. Cancer Res. 21, 1980, Seite 171). Vitamin C und E spielen ferner als Antioxidantien eine wichtige Rolle. Die gute Stabilität verschiedener Systeme im Körper wird dem Synergismus von Antioxidantien mit komplexbildenden und anderen Synergisten zugeschrieben. Bei Fetten und Ölen bedient man sich insbesondere der antioxidativen Wirkung von Tocopherol und Ascorbinsäure (H. Kläui, 14. Internation. Symposium der Commission Internationale des Industries Agricoles et Alimentaires, Saarbrücken, 8. bis 11. Oktober 1972). Es wird angenommen, daß die antioxidativen Eigenschaften der Vitamine für ihre biologische Aktivität verantwortlich sind. Vitamin E und Vitamin C reagieren sehr rasch mit freien organischen Radikalen.

Es besteht daher auch die Möglichkeit, daß die beiden Vitamine in Wechselwirkung stehen, indem Vitamin C als primäres Antioxidans wirkt und das verbleibende Vitamin-E-Radikal mit Vitamin C reagiert. Hierbei entwickelt sich wieder intaktes Vitamin E und ein Vitamin-C-Radikal bleibt zurück. Dieses kann unter gewissen Bedingungen durch ein NADH-abhängiges System enzymatisch wieder zu Vitamin C reduziert werden (Deut. Apothekerzeitung, 120. Jahrg., Nr. 2, 10.01.1980).

Es ist weiterhin bekannt, daß Cholesterin in menschlicher und tierischer Haut durch Ultraviolett-Licht zu Cholesterin-alpha-oxyd, einen als Krebserreger bekannten Stoff, umgewandelt wird. Versuche mit Mäusen haben gezeigt, daß bei Verabreichung von Vitamin E und C sowie zwei weiteren Antioxidantien sich kein Cholesterin-alpha-oxyd bildet (Pharm. Indu. 36, Nr. 3 (1974) Anschel, USA).

Vitamin E ist bekannt als Antioxidans und Schutzvitamin für Phosphorlipide der Zellmembran. Es hält die Permeabilität und Stabilität der Zellmembran aufrecht (Lucy, Ann. N.Y. Academy of Science 203, 1972, S. 4.) Es ist weiterhin bekannt, daß Vitamin E eine membranabdichtende Wirkung besitzt (F. Mittelbach und G. Bodechtel, Münchner Medizinische Wochenschrift 110 (1968) 36: 1988 - 1993). Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685 - 704 (1968) und Phelps DL Pediatrics 63 (6) S. 933 - 935 (1979).

Aus diesen Literaturstellen geht hervor, daß bei oraler Verabreichung von 200 bis 800 mg Vitamin E an Patienten für einen Zeitraum von 1 bis 4 Tagen, deren Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. clin. 33, S. 968 - 971 (1980); Natt CL. Am. J. clin. nutr. 32, S. 1359 - 1362 (1979); Gawlik G.M. Fed. Proc. 35 (3), S. 252 (1976) und Gorash L. Bieri J.G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurden, wobei eine Normalisierung der Hämolyse der Erythrocyten beobachtet wurde; vgl. Kahane I. ISR. J. med. 12 (1), S. 11 - 15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent. 74/7, S. 732 - 739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory

Nagasaki University of Medicine, Japan.

In den europäischen Patentanmeldungen 84 108 640, 85 100 749, 85 100 752, 85 101 554, 85 102 222.8 und 85 105 171 wird ferner der Einsatz von Vitamin E zur Verbesserung der Durchblutung der Extremitäten, der Peripherie des Auges, des Innenohres und des Cerebrum sowie zur Behandlung der Arteriosklerose, der Venen, von Herzerkrankungen, des Analbereichs, von Rheumaerkrankungen und der Haut sowie zum Schutz der Haut und der Schleimhaut vorgeschlagen.

Gegenstand der deutschen Patentanmeldung P 3 507 791.3 sind Vitamin-E sowie Kombinationen von Vitamin E mit anderen Wirkstoffen, die sich als Mittel, insbesondere zur Behandlung von Ekzemen, Hautflechte, Hautentzündungen, Juckreiz, Allergien, Faltenbildungen, Pigmentierungen der Haut und Haarausfall sowie Wunden, eignen. Darüberhinaus können diese Mittel als Schutz gegen ultraviolettes Licht und zur Förderung des Haarwuchses eingesetzt werden. Diese Mittel sind ferner als Hautschutzmittel bei Bestrahlungen, z. B. von Krebspatienten, geeignet.

Aus der FR-OS-2 420 973 ist ein Vitamin-E-haltiges Mittel bekannt, daß bei Erkrankungen der peripheren Gefäße, des Gehirns, des Hals-, Nasen-, Ohren-Bereichs und der Augen angewendet wird.

Aus WO-A-83/01998 ist eine pharmazeutische Wirkstoffkombination bekannt, die Vitamin A, Vitamin E, Mandelöl, Sesamöl und Olivenöl enthält.

Vitamin E kann außerdem die Zellmembran vor Oxidationen schützen und die Permeabilität sowie Stabilität der Zellmembran aufrechterhalten (Luxy Annalen N.Y., Academy of Science 203, S. 4 (1972)).

Darüber hinaus konnten membranabdichtende Eigenschaften des Vitamin E nachgewiesen werden (F. Mittelbach und G. Bodechtel, Münchener Medizinische Wochenschrift 110 (1968), 36: 1988 - 1993).

Es ist weiterhin bekannt, daß Vitamin E als Antioxidant insbesondere für Vitamin A geeignet ist. Vitamin A seinerseits ist ein gutes Schutzmittel, um die Schleimhaut vor Verhornung zu schützen. Darüberhinaus kann es als Infektionsschutz dienen. Vitamin-A-Mangel verursacht unter anderem Nachtblindheit (Hemeralopie) sowie gesteigerte Blendempfindlichkeit des Auges. Infolgedessen wurden Vitamin-A-haltige Mittel zur Behandlung der Augen entwickelt.

Aus der neueren Literatur geht hervor, daß die beiden Vitamine A und E in Wechselwirkung zueinander treten. In B. Helwig, Moderne Arzneimittel, 5. Auflage S. 1446 bis 1447 (1980) ist ausgeführt, daß der Wirkungsmechanismus des Vitamin E nur zum Teil geklärt ist. Insbesondere greift danach Vitamin E in den Umsatz und die Biosynthese von Kohlenhydraten, Eiweißkörpern, Kreatin und Nukleinsäuren ein. Die Beschleunigung der Gewebereinigung bzw. -entgiftung durch hohe Vitamin-E-Dosen wurde ebenfalls beobachtet.

Neben dem Schutz vor Oxidation des Vitamin A weisen Kombinationen der Vitamine A und E die weiteren Vorteile auf, daß Vitamin A in Gegenwart von Vitamin E besser gespeichert wird und der Vitamin-A-Serumspiegel in Gegenwart von Vitamin E normalisiert wird. Tiere mit Vitamin-E-Mangel haben einen niedrigen Serumspiegel an Vitamin A, der auch bei Zugabe von hohen Vitamin-A-Dosen niedrig bleibt.

Nach der Zugabe von Vitamin E normalisiert sich der Serumspiegel an Vitamin A jedoch wieder (vgl. hierzu Vitamin E as a useful therapeutie agent, Ayres S. Jr., Mihan R., J. Am. Acad. Dermatol. 7, (4) 521 - 5, (1982); Ayres S. Jr., Phrynoderma: Suggested Vitamin A and E Therapy (letter), Int. J. Dermatol. 22, (9) 548 - 9, (1983); Ayres S. Jr., Mihan R., Synergism of Vitamin A and E in achne vulgaris (letter), Int. J. Dermatol. 20, (9) 616, (1981); Ayres S. Jr., Dariers Disease: Update on an effective new therapy (letter), Arch. Dermatol., 119 (9) 710 (1983); Ayres S. Jr., Pittyriasis rubra pilaris controlled by synergism of Vitamin A and E (letter), J. Am. Acad. Dermatol. 5, (3) 350 - 1 (1981); Ames Sr., Factors affecting absorption, transport and storage of Vitamin A, Am. J. Clin. Nutr. 22, 934 (1969).

Diese modernen Daten der Pharmacokinetik von Vitamin C und E sollten bei der Suche nach pharmakologischen Wirkungen der Ascorbinsäure in Zukunft berücksichtigt werden. Daß darüber hinaus die Galenik eines Vitamin C-Präparates von entscheidender Bedeutung für die Pharmacokinetik der Ascorbinsäure sein kann, ist schon nach älteren Untersuchungen anzunehmen. Danach soll Vitamin C bei verzögerter Freisetzung im Darm aus einer Multivitamin-Depotform in größerer Menge das Gewebe erreichen als nach der Applikation in einer unretardierten Konfektionierung. Überraschenderweise wurde nunmehr festgestellt, daß retardiertes Vitamin C und E insbesondere in Kombination mit öllöslichen Vitaminen (z. B. Vitamin E und A) leicht herzustellen ist, wenn man diese in Neutralöl suspendiert bzw. löst. Bisher hat man Vitamin C mit Retardwirkung in Kügelchen, die unterschiedliches Freisetzungsvermögen hatten, hergestellt.

In der nicht vorpublizierten EP-A-0 127 297 ist vorgeschlagen, Vitamin- oder Mineralmischungen in eine feste Verabreichungsform zu bringen, indem man sie mit Polyoxyäthylen Sorbitan Ester vermischt und gewünschtenfalls in Gelatinekapseln einbringt. Durch diese Maßnahmen wird die Bioverfügbarkeit von Vitamin E und A erhöht. Die Bioverfügbarkeit wird definiert als Geschwindigkeit und Ausmaß der Absorption der Substanz durch den Patienten. Es findet sich kein Hinweis auf eine Retardierung der Aufnahme von Vitamin E durch den Zusatz von Polyoxyäthylensorbitan Ester.

Gegenstand der vorliegenden Erfindung ist demgemäß ein Verfahren zur Retardierung von Vitamin C und E, dadurch gekennzeichnet, daß 200 bis 800 mg Vitamin C in Neutralöl suspendiert und 150 bis 600 mg Vitamin E in Neutralöl gelöst werden und beide zusammen verkapselt werden.

Als Neutralöle kann man beispielsweise Sojabohnenöl, Olivenöl oder Erdnußöl einsetzen. Zusätzlich kann man hydriertes Sojabohnenöl, partial oder vollständig hydrierte Triglyceride oder Sojalecithin verwenden. Vorzugsweise verwendet man gesättigte Triglyceride mit 8 bis 18 C-Atomen. Ein typisches Beispiel für die erfindungsgemäßen Neutralöl-Mischungen ist ein Gemisch aus Sojabohnenöl, hydriertem Sojabohnenöl und Sojalecithin. Aus dieser Mischung wird das Vitamin C langsam im Körper resorbiert bzw. langsam freigesetzt. Dadurch wird Vitamin C mit Retardwirkung mit einem Maximum zwischen 4 und 8 Stunden hergestellt. Diese Mischung in Neutral-Öl hat den Vorteil, daß Vitamin E als öllöslicher Stoff in größeren Mengen zugesetzt werden kann, so daß es gleichzeitig resorbiert und retardiert wird. Der Plasmaspiegel des Vitamin E erreicht überraschenderweise schon bei Verabreichung von 200 I.E. maximale Werte.

Aus den Arbeiten von Hashim und Schuttringer (Am. J. Clin. Nutr. 19, 1966, 137) ist bekannt, daß bei oraler Verabreichung von 600 mg D-alpha-Tocopherol an eine Versuchsperson maximale Plasmaspiegel nach etwa 7 Stunden zu verzeichnen sind. M.K. Horwitt (Am. Soc. clin. Nutr. 40, 1984, 240 - 255) ermittelte bei Einnahme von 800 I.E. Vitamin E ein Maximum bei 7 Stunden.

Die erfindungsgemäßen Mischungen dagegen weisen bei Verabreichung von nur 200 I.E. Vitamin E ein Maximum bei ca. 6 Stunden auf. Der Vitamin E-Gehalt im Blutplasma blieb bis nahezu 12 Stunden anschließend unverändert. In der oben genannten Arbeit von Hashim und Schuttringer fiel hingegen nach Erreichen des Maximums bei 7 Stunden die Plasma-Tocopherol-Konzentration steil ab.

Die Konzentration an Vitamin C erreicht erfindungsgemäß ebenfalls ihr Maximum bei 6 Stunden, um danach allerdings steiler als die Vitamin E-Konzentration abzufallen und anschließend 12 bis 24 Stunden nahezu konstant zu bleiben. Neben den bereits bekannten synergistischen Wirkungen von Vitamin C und E-Mischungen wurde hier eine neue überraschende Retardwirkung festgestellt, die nach dem Stand der Technik nicht zu erwarten war.

Bei Verwendung von partial oder vollständig hydrierten Triglyceriden oder Mischungen mit gesättigten Triglyceriden mit 8 bis 18 Kohlenstoff-Atomen zusammen mit Sojalecithin läßt sich die Freisetzung bzw. die Retardwirkung von Vitamin C variieren.

Vitamin C kann in Form der Natrium- und/oder Calciumsalze vorliegen. Vitamin E kann in allen seinen freien Alphaformen oder als Ester zugesetzt werden. Maßgebend für die vorliegende Erfindung ist, daß Vitamin C in genügend hoher Dosierung vorhanden ist. Die Mengen liegen zwischen 200 und 800 mg Vitamin C, vorzugsweise 250 bis 600 mg pro Darreichungsform.

Die Werte für Vitamin E liegen zwischen 150 und 600 mg, vorzugsweise 200 bis 500 mg pro Darreichungsform.

In den nachfolgenden Beispielen werden typische Kombinationen von Vitamin C und E angeführt:

**Beispiel 1**

1 Kapsel enthält:

200 mg D-alpha-Tocopherol-Konzentrat
500 mg Ascorbinsäure
300 mg Sojabohnenöl
  50 mg gehärtetes Sojabohnenöl (Jodzahl Max. 3)
  10 mg Sojalecithin

**Beispiel 2**

1 Kapsel enthält:

16.500 I.E. Vitamin A als Palmitat
400 mg Ascorbinsäure
250 mg D1-alpha-Tocopherolacetat
270 mg Sojabohnenöl
  40 mg hydriertes Sojabohnenöl
  10 mg Sojalecithin

**Beispiel 3**

1 Kapsel enthält:

400 mg D-alpha-Tocopherol-Konzentrat
330 mg Ascorbinsäure
160 mg Sojabohnenöl
  10 mg Sojalecithin
  40 mg hydriertes Sojabohnenöl

**Beispiel 4**

gemäß Beispiel 3. Anstelle von Sojabohnenöl wurde Erdnußöl verwendet.

**Beispiel 5**

gemäß Beispiel 2. Anstelle von 270 mg Sojabohnenöl wurden 130 mg Sojabohnenöl und 130 mg Lebertran (Öl jecoris) verwendet.

**Beispiel 6**

gemäß Beispiel 1. 100 mg Sojabohnenöl wurden durch 100 mg Olivenöl ersetzt.

**Beispiel 7**

gemäß Beispiel 2. Anstelle von 270 mg Sojabohnenöl wurden 330 mg verwendet und 30 mg Sojalecithin anstelle von 10 mg.

**Beispiel 8**

1 Kapsel enthält:
600 mg Ascorbinsäure
300 mg Sojabohnenöl
50 mg hydriertes Sojabohnenöl
12 mg Sojalecithin

Durch das nachfolgende Beispiel 9 wird die ausgezeichnete Retardwirkung der erfindungsgemäßen Vitamin E- und Vitamin C-Präparate belegt:

**Beispiel 9**

Prüfung der Bioverfügbarkeit von Vitamin C und E. Der Versuchsbericht ist in folgende zwei Teile geteilt:
I. Gehalt und Einheitlichkeit des Gehaltes an Vitamin C und E
II. Bioverfügbarkeit, getestet an zehn freiwilligen Versuchspersonen
I.

Das Präparat EVINA ® E + C wurde auf seinen Gehalt an Vitamin E und C und die Gleichmäßigkeit des Gehaltes an Vitamin C Und E gemäß DAB Und USP Untersucht. Die Ergebnisse entsprachen der Deklarierung: Gewicht = 1582 mg ± 15 Vitamin E 219 I.E. ± 8 Vitamin C 512 mg ± 13.
Die einzelnen Werte sind den nachfolgenden Tabellen 1 bis 3 zu entnehmen.

**Tabelle 1**

EVINA ® E + C, Kapselgewicht, Mittelwerte und Standardabweichung

| Nr. | mg |
|---|---|
| 1 | 1590 |
| 2 | 1611 |
| 3 | 1586 |
| 4 | 1567 |
| 5 | 1577 |
| 6 | 1590 |
| 7 | 1600 |
| 8 | 1579 |
| 9 | 1586 |
| 10 | 1605 |
| 11 | 1553 |
| 12 | 1575 |
| 13 | 1567 |
| 14 | 1564 |
| 15 | 1565 |
| 16 | 1570 |
| 17 | 1573 |
| 18 | 1600 |
| 19 | 1590 |
| 20 | 1590 |
| Mittelwerte | 1581,9 |
| Varianz | 237,8 |
| Std. Abweichung | 15,4 |
| Min. | 1553,0 |
| Max. | 1611,0 |

**Tabelle 2**

EVINA ® E + C, Vitamin E-Gehalt, Mittelwerte und Standardabweichung

| Nr. | I.E. |
|---|---|
| 1 | 223 |
| 2 | 224 |
| 3 | 226 |
| 4 | 202 |
| 5 | 226 |
| 6 | 225 |
| 7 | 222 |
| 8 | 220 |
| 9 | 214 |
| 10 | 209 |
| Mittelwerte | 219,1 |
| Varianz | 66,5 |
| Std. Abweichung | 8,2 |
| Min. | 202,0 |
| Max. | 226,0 |

**Tabelle 3**

EVINA ® E + C, Vitamin C-Gehalt, Mittelwerte und Standardabweichung

| Nr. | mg |
|-----|------|
| 1 | 497,5 |
| 2 | 508,3 |
| 3 | 509,6 |
| 4 | 519,5 |
| 5 | 496,7 |
| 6 | 524,8 |
| 7 | 493,1 |
| 8 | 513,9 |
| 9 | 530,0 |
| 10 | 523,9 |
| | |
| Mittelwerte | 511,7 |
| Varianz | 167,7 |
| Std. Abweichung | 13,0 |
| Min. | 493,1 |
| Max. | 530,0 |

II.

**Bioverfügbarkeitsuntersuchung**

1. Ziel dieser Untersuchung ist, die Bioverfügbarkeit des neuen Vitamin E und C enthaltenden Retardpräparates zu untersuchen. Das Präparat enthielt 200 I.E. Vitamin E in natürlicher Form und 500 mg Vitamin C.

2. Materialien und Methoden

2.1. Vitamin E und C enthaltende Kapseln mit dem Handelsnamen EVINA® E + C, Zulassungs-Nr. 4715.00.00, Ch.-B. 42000302.

Gewichte, Konzentrationen und Standardabweichungen:
Gewicht = 1582 mg ± 15
Vitamin E-Gehalt = 219 I.E. ± B
Vitamin C-Gehalt = 512 mg ± 13

Die Reinheit aller Präparate wurde mittels Dünnschichtchromatographie und Photometrie untersucht.

2.2 Freiwillige Versuchspersonen

Der Versuch wurde durchgeführt mit zehn freiwilligen Versuchspersonen. Alle Versuchspersonen waren Männer. Sie wurden untersucht, um sicherzustellen, daß keine Nierenstörung vorhanden ist.

Jede der Versuchspersonen erhielt eine Kapsel EVINA ® E + C. Die Männer hatten ein Alter zwischen 25 und 72 Jahren und ein Gewicht zwischen 76 und 94 kg. Es waren sowohl Raucher als auch Nichtraucher unter den Versuchspersonen. Alle Versuchspersonen waren gesund. Die klinischen und biochemischen Untersuchungsergebnisse lagen innerhalb der Norm.

2.3. Sammeln der Blutproben

Jede Kapsel wurde am frühen Morgen mit einem Glas Wasser verabreicht. Die Blutproben wurden der Maginalvene am Arm vor der Verabreichung entnommen und 1, 2, 4, 8, 12 und 24 Stunden nach der oralen Verabreichung wiederholt. Das Blut wurde heparenisiert, zentrifugiert, das Plasma abgetrennt und bei 30°C bis zur Analyse aufbewahrt.

2.4. Analyse

Die Vitamin E-Bestimmung wurde mittels Hochdruckflüssigkeitschromatographie durchgeführt. Die Plasmaprobe wurde mit 10 %-igem NaOH alkalisch gemacht und mit Ethylacetat extrahiert. Die Probevolumen wurden auf 10 Microliter aufgefüllt.

Stationäre Phase: Lichochrosorb Si 60, Methanol als Elutionsmittel, UV-Detektor bei 285 Nanometer.

Die Standardkurve wurde mit Tocopherol in Methanol ermittelt. Die linearen Korrelationskoeffizienten wurden aufgerundet auf 0,999. Das Vitamin C wurde mit klassischen Methoden analysiert.

3. Ergebnisse:

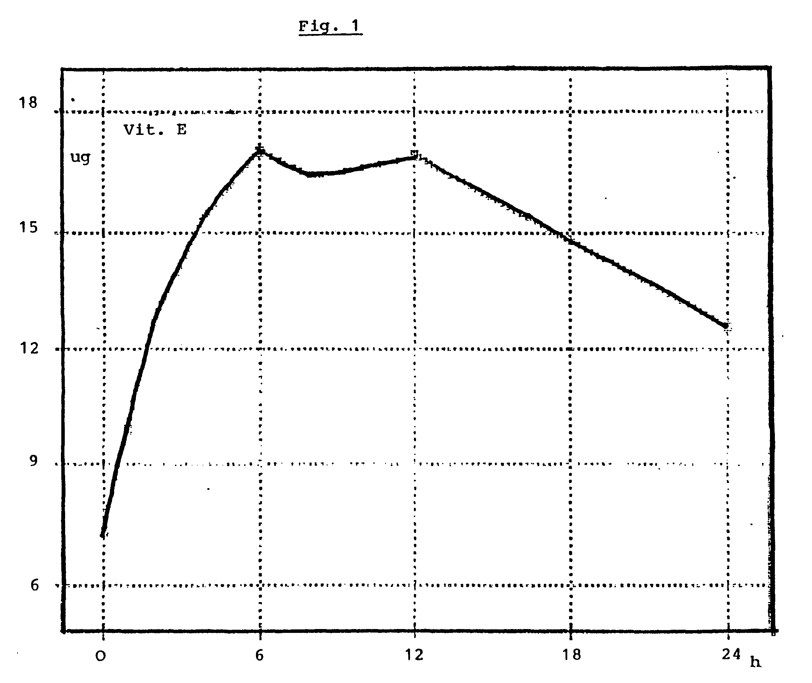

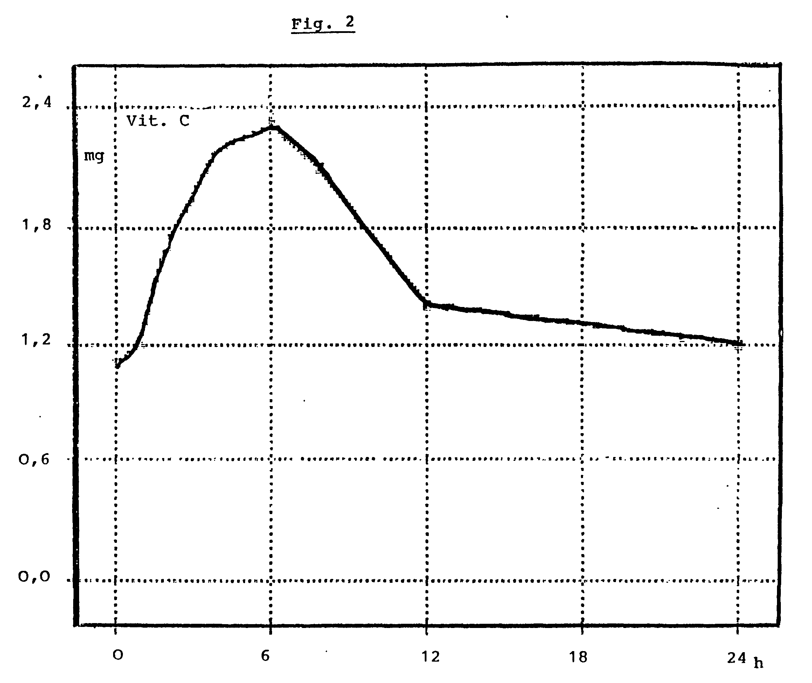

Die Analyse des Blutplasmaspiegels offenbarte signifikante Spiegel des Vitamins E und C vor der Dosierung und nach 24 Stunden. Die Ergebnisse zwischen 0 und 24 Stunden sind in den Tabellen 6 und 7 zusammengestellt; Tabelle 6: Vitamin E und Tabelle 7: Vitamin C. Die Ergebnisse sind zusätzlich in den Figuren 1 und 2 anschaulich dargestellt. Figur 1 gibt den Vitamin E-Spiegel wieder und Figur 2 den Vitamin C-Spiegel.

4. Diskussion

Die Untersuchung wurde vorgenommen, um die Bioverfügbarkeit von Vitamin E + C-Retardkapseln der Marke EVINA ® E + C zu untersuchen. Die Ergebnisse lassen sich wie folgt zusammenfassen:

EVINA ® E + C-Kapseln zeigen einen hohen Retardeffekt. Diese Ergebnisse waren erheblich besser als die in der Literatur angegebenen Werte.

Hierzu sei auf die Arbeiten von Hashim und Schuttringer (Amer. J. Clin. Nutr. 19, 1966, 137) verwiesen, wonach bei oraler Verabreichung von 600 ml D-alpha-Tocopherol ein Maximum der Plasma-Tocopherol-Konzentration nach 7 Stunden auftritt. Nach diesem Maximum fällt die Konzentration steil wieder ab.

Im Gegensatz dazu wurden bei den vorliegenden Versuchen bei Verabreichung erheblich geringerer Vitamin E-Konzentrationen nach 6 Stunden Maximalwerte erreicht, die bis zu 12 Stunden gleich blieben.

**Tabelle 6**

Vitamin E Analytik, μg/ml Mittelwert und Standardabweichung

Stunden:

| Nr. | 0 | 1 | 2 | 4 | 6 | 8 | 12 | 24 |
|---|---|---|---|---|---|---|---|---|
| 1 | 7,5 | 9,0 | 11,0 | 11,0 | 12,0 | 11,5 | 19,7 | 10,3 |
| 2 | 7,2 | 8,3 | 9,4 | 11,0 | 12,2 | 11,3 | 15,6 | 11,3 |
| 3 | 8,4 | 11,6 | 15,1 | 23,8 | 20,8 | 22,8 | 15,6 | 14,4 |
| 4 | 6,0 | 10,5 | 15,1 | 18,7 | 25,7 | 20,6 | 17,0 | 14,2 |
| 5 | 7,3 | 8,7 | 10,4 | 11,0 | 12,0 | 11,4 | 18,1 | 10,8 |
| 6 | 8,0 | 10,3 | 13,5 | 12,6 | 16,0 | 16,5 | 17,5 | 12,0 |
| 7 | 7,0 | 10,0 | 13,1 | 15,0 | 13,9 | 15,1 | 18,0 | 12,0 |
| 8 | 6,5 | 9,6 | 12,1 | 14,3 | 18,5 | 16,0 | 16,5 | 12,5 |
| 9 | 7,8 | 10,0 | 12,1 | 17,0 | 16,8 | 18,0 | 15,5 | 12,8 |
| 10 | 7,4 | 11,0 | 15,2 | 20,2 | 22,1 | 21,0 | 16,0 | 14,3 |
| | | | | | | | | |
| Mittelwerte | 7,3 | 9,9 | 12,7 | 15,5 | 17,0 | 16,4 | 16,9 | 12,5 |
| Varianz | 0,5 | 1,1 | 4,3 | 19,4 | 22,5 | 17,7 | 1,9 | 2,2 |
| Std.-Abwei. | 0,7 | 1,0 | 2,1 | 4,4 | 4,7 | 4,2 | 1,4 | 1,5 |
| Min. | 6,0 | 8,3 | 9,4 | 11,0 | 12,0 | 11,3 | 15,5 | 10,3 |
| Max. | 8,4 | 11,6 | 15,2 | 23,8 | 25,7 | 22,8 | 19,7 | 14,4 |

**Tabelle 7**

Vitamin C Analytik, mg/100 ml Mittelwert und Standardabweichung

Stunden:

| Nr. | 0 | 1 | 2 | 4 | 6 | 8 | 12 | 24 |
|---|---|---|---|---|---|---|---|---|
| 1 | 0,6 | 0,8 | 1,4 | 2,1 | 2,1 | 1,8 | 1,2 | 0,8 |
| 2 | 1,3 | 1,4 | 1,8 | 2,8 | 2,9 | 2,5 | 1,5 | 1,5 |
| 3 | 1,0 | 1,1 | 1,5 | 1,8 | 1,9 | 2,1 | 1,4 | 1,1 |
| 4 | 1,1 | 1,2 | 1,6 | 1,9 | 2,1 | 2,3 | 1,2 | 1,0 |
| 5 | 0,9 | 1,1 | 1,3 | 1,6 | 1,6 | 1,5 | 1,4 | 1,0 |
| 6 | 1,1 | 1,4 | 2,0 | 2,6 | 2,5 | 2,3 | 1,7 | 1,5 |
| 7 | 1,2 | 1,3 | 1,9 | 2,4 | 2,4 | 1,9 | 1,3 | 1,1 |
| 8 | 1,3 | 1,3 | 2,1 | 2,8 | 2,7 | 2,4 | 1,3 | 1,3 |
| 9 | 1,2 | 1,4 | 1,7 | 2,0 | 2,8 | 2,1 | 1,5 | 1,3 |
| 10 | 1,0 | 1,2 | 1,6 | 1,9 | 2,1 | 1,7 | 1,4 | 1,1 |
| | | | | | | | | |
| Mittelwerte | 1,1 | 1,2 | 1,7 | 2,2 | 2,3 | 2,1 | 1,4 | 1,2 |
| Varianz | 0,0 | 0,0 | 0,1 | 0,2 | 0,2 | 0,1 | 0,0 | 0,1 |
| Std.-Abwei. | 0,2 | 0,2 | 0,3 | 0,4 | 0,4 | 0,3 | 0,2 | 0,2 |
| Min. | 0,6 | 0,8 | 1,3 | 1,6 | 1,6 | 1,5 | 1,2 | 0,8 |
| Max. | 1,3 | 1,4 | 2,1 | 2,8 | 2,9 | 2,5 | 1,7 | 1,5 |

# EP 0 176 772 B1

## Patentansprüche

1. Verfahren zur Retardierung von Vitamin C und E, dadurch gekennzeichnet, daß 200 bis 800 mg Vitamin C in Neutralöl suspendiert und 150 bis 600 mg Vitamin E in Neutralöl gelöst werden und beide zusammen verkapselt werden.

2. Verfahren gemäß Anspruch 1, dadurch gekennzeichnet, daß als Neutralöl Sojabohnenöl, Olivenöl, Erdnußöl oder Rüböl sowie gegebenenfalls zusätzlich hydriertes Sojabohnenöl, partial oder vollständig hydrierte Triglyceride oder Sojalecithin eingesetzt werden.

3. Verfahren gemäß einem der Ansprüche 1 oder 2, dadurch gekennzeichnet, daß man 250 bis 600 mg Vitamin C und 200 bis 500 mg Vitamin E pro Kapsel einsetzt.

4. Verfahren gemäß einem der Ansprüche 1 bis 3, dadurch gekennzeichnet, daß man zusätzliche fettlösliche Vitamine, Lebertran, Nicotinsäureamid und Vitamine der B-Reihe verwendet.

5. Verfahren gemäß einem der Ansprüche 1 bis 4, dadurch gekennzeichnet, daß man Vitamin C in Form des Natriumund/oder Calciumsalzes einsetzt.

6. Verwendung von 200 bis 800 mg Vitamin C und 150 bis 600 mg Vitamin E suspendiert bzw. gelöst in Neutralöl zur Retardierung der Vitamine C und E.

## Claims

1. A process for sustaining the release of vitamins C and E, characterized in that from 200 to 800 mg of vitamin C are suspended in neutral oil and from 150 mg to 600 mg of vitamin E are dissolved in neutral oil and both together are encapsulated.

2. The process according to claim 1, characterized in that soybean oil, olive oil, peanut oil or rape-seed oil are employed as neutral oil and, if so desired, hydrogenated soybean oil, partially or fully hydrogenated triglycerides or soybean lecithin are additionally employed.

3. The process according to any one of claims 1 and 2, characterized in that from 250 to 600 mg of vitamin C and from 200 mg to 500 mg of vitamin E are employed per capsule.

4. The process according to any one of claims 1 to 3, characterized in that additionally fat-soluble vitamins, (cod) liver oil, nicotinic amide and vitamins of the B series are employed.

5. The process according to any one of claims 1 to 4, characterized in that vitamin C is employed in the form of the sodium and/or calcium salt.

6. Use of from 200 to 800 mg of vitamin C and of from 150 mg to 600 mg of vitamin E suspended or dissolved, respectively, in neutral oil for the sustained release of the vitamins C and E.

## Revendications

1. Procédé pour le retardement des vitamines E et C, caractérisé en ce qu'on met 200 à 800 mg de vitamine C en suspension dans une huile neutre, on dissout 150 à 600 mg de vitamine E dans une huile neutre, et on encapsule ces deux produits ensemble.

2. Procédé selon la revendication 1, caractérisé en ce que l'on emploie comme huiles neutres de l'huile de soja, de l'huile d'olive, de l'huile d'arachide ou de l'huile de colza ainsi qu'éventuellement en plus d'huile de soja hydrogénée, des triglycérides partiellement ou totalement hydrogénés ou de la lécithine de soja.

3. Procédé selon les revendications 1 ou 2, caractérisé en ce que l'on emploie 250 à 600 mg de vitamine C et 200 à 500 mg de vitamine E par capsule.

4. Procédé selon l'une des revendications 1 à 3, caractérisé en ce que l'on emploie des vitamines additionnelles solubles dans les corps gras, l'huile de foie de morue, l'amide d'acide nicotinique et les vitamines de la série B.

5. Procédé selon l'une des revendications 1 à 4, caractérisé en ce que l'on emploie la vitamine C sous forme de sels de sodium ou de calcium.

6. Emploi de 200 à 800 mg de vitamine C et de 150 à 600 mg de vitamine E en suspension ou en solution dans une huile neutre pour le retardement des vitamines C et E.

Fig. 1

Fig. 2

EP 0 176 772 B1